# EUROPEAN PATENT SPECIFICATION

(11) **EP 2 688 647 B1**
(45) Date of publication and mention of the grant of the patent: **09.09.2015**
(21) Application number: 12715177.7
(22) Date of filing: 26.03.2012
(51) Int. Cl.: A61N 5/10

(54) **APPARATUS FOR ELECTRONIC BRACHYTHERAPY**
VORRICHTUNG FÜR EINE ELEKTRONISCHE BRACHYTHERAPIE
APPAREIL POUR LA CURIETHÉRAPIE ÉLECTRONIQUE

(30) Priority: 24.03.2011 EP 11159620
(43) Date of publication of application: 29.01.2014
(73) Proprietor: Koninklijke Philips N.V., 5656 AE Eindhoven (NL); Philips Intellectual Property & Standards GmbH, 20099 Hamburg (DE)
(72) Inventor: RIBBING, Carolina, 5656 AE Eindhoven (NL)
(74) Representative: Steffen, Thomas
(86) International application number: PCT/IB2012/051418
(87) International publication number: WO 2012/127455

(56) References cited:
- EP-A1- 1 314 452
- EP-A1- 1 445 002
- EP-A2- 1 745 822
- WO-A1-01/13060
- US-A- 5 092 834
- US-A1- 2003 018 232

## Description

### FIELD OF THE INVENTION

The present invention relates to a system, apparatus, and computer program product for brachytherapy guidance, wherein a radiation source is to be placed or moved in relation to tissue, especially tumorous tissue, in order to provide a radiation dose to tissue, especially according to a radiation therapy plan, e.g. a therapy plan envisaging several therapy fractions.

### BACKGROUND OF THE INVENTION

Radiation source placement technology in brachytherapy usually is based on a system using ultrasound imaging or on a system using X-ray or computed tomography. Integrated into an applicator or a catheter, such a system for correct placement or localization is aimed to provide a correct radiation dose to tumorous tissue and to prevent excessive radiation of healthy tissue.

In treatments of e.g. prostate cancer, breast cavities, cervix cancer, tumors of the mouth and pharynx, lung cancer or liver cancer, there are two radiation therapy concepts commonly applied: the isotopic brachytherapy and the electronic brachytherapy. The main differences consist in the radiation energy which is considerably lower in electronic brachytherapy, providing radiation energy of e.g. max. 50 keV, (the radiation source can be turned off also) as well as in the treatment possibilities: in electronic brachytherapy, x-ray facilities as well as standard operation rooms might be used (short range and low mean energy of the radiation), which is not possible in isotopic brachytherapy, especially in so called high dose rate (HDR) brachytherapy. Generally, in isotopic brachytherapy, the radiation sources usually are millimeter-sized seeds of radioactive isotopes, like e.g. the iridium isotope Ir-192, providing radiation energy in the range of 350 keV.

For both radiation therapy concepts, the exact position of the applicator as well as the exact position of the seed or any other radiation source is vital. Usually, an applicator can be placed under real-time image guidance based on e.g. ultrasound (US) or x-ray, or it can be imaged after placement (based on e.g. computer tomographic CT techniques) or placed based on previously registered images, wherein the seed is pulled (where applicable robotically) through the applicator or through interstitially implanted catheters. Problems appear when it is required to displace the applicator, not least because one or several additionally required CT scans of the treatment area, or when the applicator moves between fractions, so that radiation exposure of the patient might be disadvantageously high. For example, in isotopic brachytherapy, imaging is done before each fraction, wherein during the course of therapy e.g. about ten CT scans can be required to check if the applicator did not move since the last fraction.

In particular, in HDR brachytherapy, basing the placement on pre-registered images or on real-time ultrasonic imaging severely limits the placement accuracy due to limited resolution or patient movement, organ movements (e.g. bowel, uterus), or tissue deformation (e.g. tissue compression by the applicator, swelling, etc). Further, when using x-ray or CT placement guidance, as suggested above, care must be taken to keep down the dose delivered through the imaging and CT time is often scarce. Also, x-ray imaging has limited soft tissue contrast, making it difficult to discern tumorous tissue, fat, or muscle. Other problems usually met in context with radiation therapy are missing information on local tissue characteristics, i.e. exact localization of various soft tissues or determination of the local tumor grade resp. the microvascular environment, the tissue pH, the tissue oxygenation or any extent of necrotic regions is not possible. Also, e.g. in treatment of lumpectomized breast cancer, the tumor cavity being filled by air or liquid might result in different dose distributions, complicating a therapy according to a specific dose plan.

Furthermore, identification of the target volume and critical organ margins relative to the implanted applicators and controlling and compensating for patient motion is often difficult. But adherence to the dose plan which might be created based on tumor stage, tumor size, imaging, biopsy, and potential surgical information is vital in any form of brachytherapy. Thus, all these items lead to large safety margins in order to ensure dose delivery to all areas of malignant and potentially malignant tissue at the expense of side-effects to the patient caused by radiation exposure of healthy tissue.

Recently designed devices for tissue monitoring in brachytherapy are based on e.g. optical observation systems facilitating the identification of the tissue region which is to be irradiated. In particular, such optical systems can be arranged within a catheter and rely on e.g. optical coherence tomography (OCT). More details about such optical analyzing applications can be found e.g. in the US 2008/0298548 A1 or US 2005/0187422 A1. It is however possible to use this visualization technology, not to achieve the function of an exact absolute position determination, but to analyze any data related to tissue characteristics, e.g. in combination with generation of a mark of the tissue region to be treated. Furthermore, magnetic navigation is a known technique for determining the position of a radiation source, which is disclosed in the WO 2007/083310 A2 or WO 2009/053897 A1. It is possible to use this technology to position the applicator in relation to tissue which is to be treated. In this way, a positioning of an x-ray source in relation to the tissue is possible.

The European patent application EP1314452A1 discloses a brachytherapy apparatus comprising guiding means for placing radioactive seeds using needles and optical means for sensing the position of the needles.

### SUMMARY OF THE INVENTION

It is an object of the present invention to provide a brachytherapy guidance system for acquiring exact position data of a radiation source within a body in order to reduce dosimetry deviations, e.g. due to the influence of applicator positioning and applicator attenuation. It is a further object of the present invention to provide an electronic brachytherapy guidance system with at least one position sensor for improving accuracy of position and movement information of a radiation source relative to the irradiated tissue. Also, it is an object of the present invention to provide a brachytherapy system for sparing healthy tissue of the patient as well as treatment personnel, and to facilitate radiation therapy, also in context with intra-fraction monitoring for a therapy plan envisaging several fractions or in context with adaptive re-planning. In other words, it is an aim of the present invention to improve source placement in vivo and thus achieve a higher precision in the administered dose and carry out a radiation therapy more effectively.

At least one of these objects is achieved by an apparatus as claimed in claim 1, an applicator device as claimed in claim 8, a system for integrated guidance for positioning a radiation source and/or its applicator within a body as claimed in claim 10, and a computer program product for positioning a radiation source. Thereby, the miniature radiation source can be provided e.g. in the form of a miniature x-ray tube or a radioactive source, e.g. Ir-192. Further, means for local tissue sensing are incorporated in the system.

Accordingly, a brachytherapy guidance system, especially a low energy electronic brachytherapy guidance system, with one or more position sensors, especially tracking sensors, is provided for conducting a radiation therapy according to a dose plan, wherein an apparatus for guiding a radiation probe is provided which is arranged to provide an arrangement for combining several sensing techniques for acquiring position data as well as tissue data, especially with differing resolutions depending on a respective sensor. I.e., interventional guidance is provided by at least one position sensor adapted to a single of electromagnetic (EM) tracking and fiber Bragg grating (FBG) any fiber-optical shape sensing and localization technique e.g. based on Rayleigh scattering or other sensing techniques, especially optical sensing techniques, or a combination of these localization techniques, in order to improve source resp. tube placement as well as tissue treatment. By integrating guidance functions in a brachytherapy system, especially into an applicator and/or a catheter, the problems regarding lack of real-time imaging and imprecise radiation dose applied to tumorous tissue can be reduced or mitigated. Thereby, it has been found that in context with electronic brachytherapy providing much lower radiation energy, the radiation dose can be given with higher spatial resolution in the tissue, so that a surgeon can benefit of a more precise guiding in order to enhance the therapy plan and reduce tissue exposure. Also, a surgeon can benefit of more precise repeatable placement. In particular, it has been found that the exact positioning is more important for low-energy sources (e.g. mini-x-ray tubes) than for the traditional sources (e.g. Ir source, providing energies of more than 350keV), especially because low energy allows for higher-resolution dose-painting. In non-implantable brachytherapy (meaning electronic and HDR isotopic), the radiation source is used with an applicator, which is placed first. It has been found that, in contrast thereto, a mini-x-ray-tube, especially directly on a probe, e.g. in the form of an endoscope, but thinner, can omit the use of an applicator, and thus call for accurate placement of the source directly instead of the applicator. Thereby, guiding means provided by an apparatus as claimed in claim 1 can be designed in the form of a probe, especially with the functionality of a thin endoscope, so that direct placement of the source can be realized, wherein only small incisions are required.

The brachytherapy guidance system can also be used in vessel applications, although the requirement of more precise guidance rather originates from brachytherapy since in vessel applications, guidance is given by the vessel itself in two dimensions and only the third dimension along the vessel has to be adjusted. Likewise, the requirement of more precise tissue monitoring rather originates from brachytherapy than from vessel applications, since a shorter radiation range in tissue is needed because the target tissue is closer.

Alternatively, or additionally, at least one tissue sensor resp. environment sensor for determining local tissue characteristics can be provided, e.g. based on spectroscopy measurements. By integrating tissue sensing functions in a brachytherapy system, the problems regarding low soft tissue contrast or missing information on the character of the irradiated tissue can be reduced or even mitigated.

Thereby, the guidance function can be based on fiber Bragg grating (FBG). In particular, a FBG sensor is a distributed Bragg reflector inscribed in a segment of an optical fiber. By periodic modification of the refractive index of the fiber core, a dielectric mirror is achieved. The FBG sensor transmits all wavelengths except the reflected so-called Bragg wavelength and a certain bandwidth around it, wherein the Bragg wavelength of a certain FBG sensor changes with strain and temperature. Therefore, FBG sensors are often used as sensors of strain, temperature or other measures translated into these. A large number of FBG sensors can be multiplexed in (along) a same fiber using optical frequency domain reflectometry in order to obtain measurements along a whole catheter.

A guidance function of the brachytherapy system can also be based on electromagnetic (EM) position sensors resp. electromagnetic (EM) tracking sensors, solely or in addition to fiber Bragg grating (FBG). Generally, electromagnetic (EM) tracking can be carried out by placing a patient in a low-frequency electromagnetic field and locating spatial localization means on or within the patient's body. Localization data can be acquired by the position, where applicable the real-time position, of these spatial localization means, e.g. a coil or a multitude of small coils, which can be incorporated into the radiation source and/or its applicator, especially at a distal end of the applicator. EM guidance systems are available from e.g. Traxtal Inc and SuperDimension Inc. Prior art includes e.g. US 2002/0143317 and A. ERNST et al.: Electromagnetic guidance for transbronchial biopsy of peripheral lung lesions: navigational bronchoscopy, CTSNet. The coils can function as position sensors and provide feedback on position (x, y, z) and movement (yaw, pitch, roll). Relating to the brachytherapy application of the present invention, the position sensor can be such a coil for EM tracking, e.g. placed at the distal end or tip of a radiation source.

Thus, the position of the radiation source resp. the guidance of the radiation source, e.g. a miniature x-ray tube, can be provided by FBG sensors or EM tracking, as mentioned above, but alternatively also by optical spectroscopy, optical coherence tomography (interferometric imaging using near-IR), and/or GPS or dGPS. These methods have different spatial resolution and may be used single or in combination. In other words, only one of these systems may be used, or several or all of them in combination, e.g. FBG for accuracy better than 1 mm, EM tracking for accuracy in the region above 1 mm, and dGPS for cm accuracy. Other tracking technologies are possible. Further, position detection and/or tissue analyzing can be carried out with respect to a field of view which is different than the field of view of the radiation source, especially in case there are provided more than one tissue sensor resp. position sensor. Thus, the brachytherapy applicator can be arranged for both probing the tissue which is to be irradiated and probing the local microenvironment.

Thereby, the localization data provided by these localization techniques can be used to confirm the placement of the applicator and/or the source relative to the therapy plan or to improve placement accuracy. An on-probe localization system can also be used for intra-fraction monitoring for confirmation of source/applicator position or adaptive re-planning. The tracing data can be stored and used to refine future treatment plans and for future correlation with long-term treatment outcome. The data output from the guidance system can be analyzed and displayed as is or combined into real-time images or projected onto pre-registered images. That is to say, when using the above described techniques for radiation source guidance, the data collected can be used for different purposes. First, spectral, ultrasound, EM, and FBG data can be used to position the miniature x-ray source and/or its applicator, before treatment or during treatment for adaptive re-planning. Second, after placement, data collected before, during, and after irradiation with the radiation source may be used to plan following fractions, and to assess e.g. amount of tissue necrosis or fast radiation toxicity effects and changes in tissue perfusion indicative of delivered dose and tissue response to irradiation, i.e. treatment monitoring.

According to a first aspect, first tissue sensing means for generating tissue data related to tissue characteristics can be provided and arranged for providing the tissue data to a position control device and/or the power control device of a brachytherapy system in order to analyze tissue, especially tissue to be treated. The first tissue sensing means can be composed of at least one sensor of the group comprising a pH sensor, an O₂ sensor, an optical spectroscopy sensor and an optical coherence tomography sensor. Thereby, reflectance spectroscopy as well as fluorescence detection can be used to detect / image e.g. inherent tissue fluorescence or previously administered tumor staining, e.g. for intraoperative or lumpectomy use of the system. Thus, the brachytherapy applicator can be arranged for both position sensing and probing the local microenvironment. Further, the first position sensing means can be composed of at least one sensor of the group comprising a fiber BRAGG grating (FBG) sensor, an optical spectroscopy sensor, an optical coherence tomography sensor and a GPS sensor. The apparatus can further be arranged for providing the first position sensing means in conjunction with at least one sensor of the group comprising an FBG sensor, an optical spectroscopy sensor, an optical coherence tomography sensor, an electromagnetic (EM) sensor, a pH sensor, an O₂ sensor, and a GPS sensor.

In other words, in addition to a guidance function, a tissue monitoring function, especially a kind of photonic needle function can be integrated in an electronic brachytherapy system, wherein radiation resp. light is provided to the tissue area and reflected back to the applicator resp. to any analyzing apparatus *ex vivo,* i.e. outside the applicator resp. catheter, the light being supplied to the tissue area e.g. by means of optical fibers. Likewise, a light source can be arranged outside the applicator resp. catheter. Thereby, acquiring tissue data can simply be achieved by providing guiding means which are arranged for housing optical sensors as well as pH sensors and/or O₂ sensors, wherein an *ex vivo* arrangement can ensure an applicator to be provided with small dimensions resp. diameter.

Various optical techniques are of interest for *in vivo* characterization in conjunction with brachytherapy. These include optical spectroscopy, optical coherence tomography, fluorescence and luminescence. Prior art includes e.g. WO 2009/050667 and WO 2009/109879. Accordingly, it has been recognized that by integrating a photonic needle function into the brachytherapy system, soft tissue contrast and high resolution real-time information or even imaging can be provided, allowing for improved sparing of organs at risk as well as possibilities to irradiate certain tumor volumes with a higher dose. Thereby, the photonic needle function can denote reflectance spectroscopy using visible to IR wavelengths and potentially fluorescence spectroscopy using UV. The excitation light can be provided via an optical fiber from a white light source *ex vivo.* The reflected light, which is also transmitted through the irradiated tissue, can be collected via an optical fiber and the analysis can be done with a grating spectrometer *ex vivo.* Excitation and reflected radiation may be transmitted through the same or through different optical fibers. The excitation radiation can be pulsed or continuous. Thereby, the detected spectrum varies as a function of tissue type, e.g. varies with fat content, tissue perfusion, and is different for tumorous and non-tumorous tissue. In a simplified version, it is possible to excite only at selected wavelengths using e.g. light emitting diodes (LEDs) and detecting only certain relevant wavelength intervals without using a spectrometer. The fiber(s) for the reflectance spectroscopy can be integrated in or at the radiation source or in or at an applicator. The detected spectra can be analyzed or displayed as is, e.g. using pattern recognition techniques, or in combination with or mapped onto real-time or pre-registered images. Detected spectra collected during photonic needle movement/stepping may even be used for spectra imaging.

According to a second aspect which can be combined with the above first aspect, the first position sensing means including its optical fiber can be arranged within guiding means, at least partially adjacent to the radiation source. Thereby, the expression at least partially adjacent implies that the sensing means can be arranged both next to the radiation source or (partially) within the radiation source. The first position sensing means can further be arranged at or at least proximate to the distal end of the guiding means. The apparatus can further comprise second position sensing means in the form at least one electromagnetic (EM) tracking sensor arranged within the guiding means. The second position sensing means can be arranged to provide the position data to the position control device and/or power control device of the brachytherapy system in order to position the radiation source within the body. The first and second position sensing means can further be arranged in such a way that both position data of the radiation source and the position data of the distal end can be provided. Thereby, the first and second position sensing means can provide different spatial resolution, e.g. in the range of cm, mm, or sub-mm accuracy. This provides the advantage of generating position data with respect to specific purposes.

According to a third aspect which can be combined with any one of the above first and second aspects, the first tissue sensing means can be arranged within the guiding means, at least partially adjacent to the radiation source, especially at or at least proximate to the distal end of the guiding means. The apparatus can further comprise second tissue sensing means in the form of at least one ultrasonic (US) probe which can be arranged within the guiding means. The second tissue sensing means can be arranged to provide the tissue data to the position control device and/or power control device of the brachytherapy system in order to analyze tissue, especially tissue to be treated. Thereby, the first tissue sensing means can be arranged to probe the tissue which is to be irradiated, and the second tissue sensing means can be arranged to probe local environment, or vice versa. That is to say, the first and second tissue sensing means have different fields of view, in order to provide a capacious resp. extensive impression of the treatment area to the surgeon. Further, the first and second tissue sensing means can be arranged in such a way that both tissue data of tissue to be irradiated by the radiation source and tissue data of tissue encompassing the tissue to be irradiated can be provided, especially with different resolution. Thereby, a surgeon can get an overview of the area in which the therapy is to be conducted, also during treatment, in order to ensure that sensitive tissue is not radiated excessively.

In other words, alternatively or in addition, for sensing of the tissue characteristics, a miniature ultrasound transducer (US probe) can be integrated in the brachytherapy system to provide soft tissue contrast (e.g. intra vascular ultrasound type). Thereby, regions of necrotic tissue and certain tumorous tissue types can be detected or imaged, as well as certain organ margins and gas. The output signals can be used or interpreted as is or integrated with real-time or pre-registered images from external imaging. Further, sensing of the tissue characteristics can be provided by use of optical spectroscopy, optical coherence tomography, pH meters, and/or O₂ meters. The output from these tissue sensors can be used for feedback to adapt the dose plan or even to monitor the treatment in real-time. For example, the US probe can provide information on organ margins, gas, necrotic tissue, and spectroscopy / tomography can potentially detect bleedings or characterize tumor tissue. Data on pH and O₂ level can give information on tissue/tumor radiosensitivity and can therefore be used for dose adaption.

Thus, the at least one tissue sensor can be a miniature US probes, an optical spectroscopy sensor, a sensor for optical coherence tomography, a pH meter, and/or an O₂ meter. Other sensing technologies are possible. Further versions of sensors can include several chips for ultrasound mounted around the radiation source, around its applicator, around high voltage cabling or optical fibers. The same reasoning can be applied to the optical spectroscopy part, with several fibers or several output/input points positioned e.g. around the x-ray source or its applicator. The individual components may be arranged in various ways including all or some of the functions suggested by the present invention.

According to a fourth aspect which can be combined with any one of the above first, second and third aspects, the radiation source can be arranged within a radiation source guiding means, and the first position sensing means can be arranged within a position sensor guiding means which is provided adjacent to the radiation source guiding means. The first tissue sensing means can be being arranged within a tissue sensor guiding means which can be provided adjacent to the radiation source guiding means, so that the guiding means can provide a channel-like arrangement with each channel-like guiding means being arranged adjacent to at least one other of the channel-like guiding means. Thereby, the expression adjacent to implies that the guiding means can be arranged next to each other respectively in such a way that they directly contact each other, but also in such a way that there is a gap there between or any compensating material. Thereby, an arrangement can be provided which is flexible in view of providing different sensors with different sensor techniques or resolution. Also, one or several guiding means can be provided in such a way that the applicator possesses e.g. a specific stiffness resp. rigidity, or a specific elasticity.

According to a fifth aspect which can be combined with any one of the above aspects, the first tissue sensing means can be at least composed of an optical spectroscopy sensor using visible to infrared (IR) wavelength, and excitation light can be provided from a light source ex vivo via first optical fiber means. Reflected light can be provided via the first or via second optical fiber means. The apparatus can be connected to a grating spectrometer ex vivo for analyzing reflected light. Thereby, analyzing tissue data can be done effectively and by techniques which can be handled quite easily.

According to a sixth aspect which can be combined with any one of the above aspects, the second tissue sensing means can be provided in the form of at least one optical fiber which can be integrated, at least partially, in the radiation source within the radiation source guiding means, or which can be integrated at the radiation source within at least one of the guiding means. Alternatively, or in addition, the second tissue sensing means can be provided in the form of at least two chips for ultrasonic (US) detection, and the chips can be mounted around the radiation source and/or around the optical fiber and/or along the guiding means. Thereby, not only the characteristics of tissue encompassing the distal end of an applicator, but also tissue along the longitudinal extension of the applicator can be analyzed.

The above described techniques for radiation source guidance provide data that can be used for different purposes. Firstly, spectral, ultrasound, EM, and FBG data can be used to position the radiation source and/or its applicator, before treatment or during treatment for adaptive re-planning. Secondly, after placement, data collected before, during, and after irradiation with the radiation source may be used to plan following fractions and to monitor treatment. The output from these position or tissue sensors can be used to for accurate source placement relative to the target tissue, to monitor the treatment in real-time or for feedback to adapt the dose plan of current or future fractions.

Of course other guiding and tracking options can be used. For example, arrangement of the radiation source could be specifically adapted to the tissue area or to the shape of the applicator, and any position and/or tissue sensors could be arranged for optimal data acquisition.

It shall be understood that the apparatus of claim 1, the applicator device of claim 8, the method for positioning a radiation source, the system of claim 10 and the storage medium or distribution medium including a computer program product of claim 12 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a schematic block diagram of a system for electronic brachytherapy according to the invention, incorporating means for localization and means for monitoring;
Fig. 2 shows a schematic drawing of a brachytherapy applicator according to a first embodiment, incorporating two means for localization;
Fig. 3 shows a schematic drawing of an applicator according to a second embodiment, incorporating two means for localization as well as means for monitoring, and
Fig. 4 shows a schematic drawing of an applicator according to a third embodiment, incorporating means for localization as well as two means for monitoring.

### DETAILED DESCRIPTION OF EMBODIMENTS

In the following embodiments, an enhanced brachytherapy apparatus and system for exact radiation dose delivery is proposed where at least position data is generated by means of at least one position sensor and wherein additionally, tissue data is generated by means of at least one tissue sensor. The sensors can be based on e.g. optical sensing techniques.

According to the embodiments, a flexible brachytherapy system for generating position and/or tissue data before, during and/or after irradiation is provided, wherein the data can be used e.g. to plan following fractions of a therapy plan scheduling several fractions. Hence, the system is adapted to re-planning of brachytherapy. I.e., at least two different sensors are provided so that position data and/or tissue data can be provided by an applicator device in order to monitor treatment progress or to plan treatment according to a dose plan.

In the following, three embodiments using different sensors resp. a different combination of sensing techniques are described.

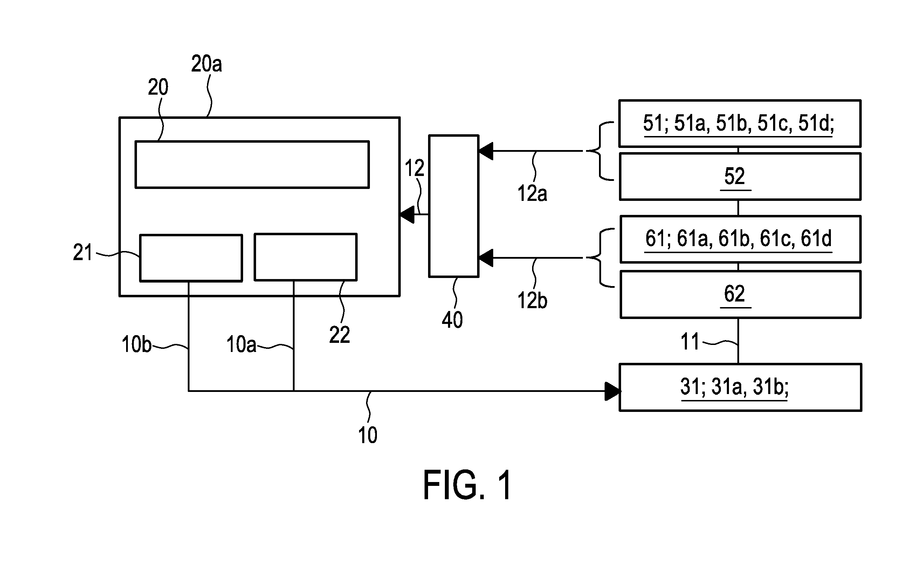

Fig. 1 shows a schematic block diagram of a system for electronic brachytherapy according to the invention, incorporating means for localization and means for monitoring. A radiation source 31 is in communication with a brachytherapy apparatus 20, e.g. in the form of a workstation, by means of a first connection 10, wherein the first connection 10 links the radiation source 31 to a position control device 22 via a position control connection 10b and to a power control device 21 via a power control connection 10a. The brachytherapy apparatus 20 as well as the position control device 22 and the power control device 21 can be provided in a housing 20a. A first and a second tissue sensor 51, 52 as well as a first and a second position sensor 61, 62 can be linked among each other by a second connection 11. Alternatively, the second connection 11 can be provided between only two of the sensors 51, 52, 61, 62, without any link to the radiation source. The second connection is provided in order to enable the brachytherapy system to correlate data from a specific sensor with data from another sensor, i.e., in order to enable a handover between the sensors 51, 52, 61, 62. In particular, such a handover may provide an enhanced guiding and monitoring function in context with different spatial resolution and/or different fields of view of the sensors. The sensors 51, 52, 61, 62 are linked to the brachytherapy apparatus 20 via a third connection 12, wherein by means of the third connection 12, also the data resp. therapeutic parameters of a brachytherapy dose plan 40 can be taken into account. With this technology, a position feedback can be provided to the brachytherapy apparatus 20 via a position feedback connection 12b. At the same time, a tissue/environment feedback can be provided to a brachytherapy apparatus 20 via a tissue feedback connection 12a in order to provide power and position settings to the radiation source 31 in accordance with the dose plan 40. The connections can be wired connections or wireless connections, e.g. based on commonly used wireless transmission techniques. The dose plan 40 may or may not be adjusted during treatment. If not adjusted during treatment, in case there are several fractions, the data from guidance sensors 61, 62 and monitoring sensors 51, 52 can be used to plan the next/remaining fraction(s) of the radiotherapy. Each sensor 61, 62, 51, 52 can provide data with a specific accuracy resp. resolution.

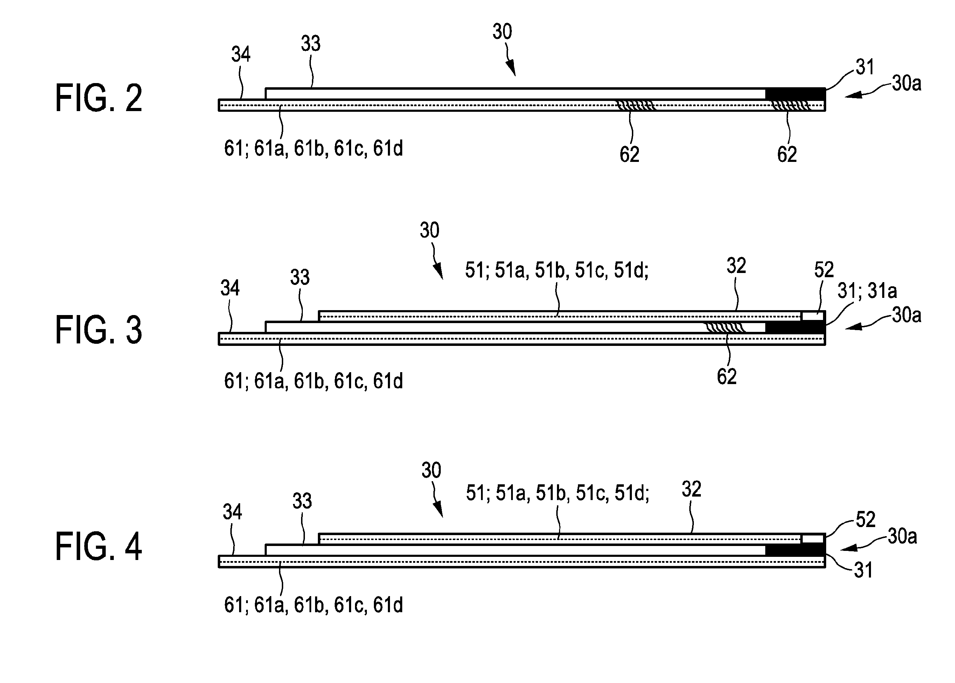

Fig. 2 shows a schematic drawing of a brachytherapy applicator 30 according to a first embodiment, incorporating two means for localization. Thereby, an applicator device can be provided in the form of at least one catheter resp. catheter-like device and/or in the form of an applicator. The radiation source 31 is placed at a distal end 30a of the applicator 30, especially within radiation source guiding means 33. The means for localization are both provided in position sensor guiding means 34 adjacent to radiation source guiding means 33. In particular, a first position sensor 61 is provided in the form of a fiber 61a, e.g. a FBG fiber, extending along the position sensor guiding means 34, and a second position sensor 62 is provided in the form of an EM coil at the distal end 30a of the applicator 30, wherein in this embodiment, a second EM coil is provided around the first position sensor 61, especially within the applicator 30 and at a distance to the distal end 30a of about 1/3 of the absolute length of the applicator 30. One or several second position sensors 62 can be provided along the extension of the applicator 30 also. Alternatively, the first position sensor 61 can be provided in the form of an optical spectroscopy sensor 61b, an optical coherence tomography sensor 61 c, or a GPS sensor 61 d. The radiation source 31 may be designed and placed to emit preferentially in the forward direction corresponding to the extension of the applicator 30 or essentially isotropically.

Fig. 3 shows a schematic drawing of an applicator 30 according to a second embodiment, incorporating two means for localization as well as means for monitoring. The radiation source 31 is placed as shown in Fig. 1. The means for localization are provided in the position sensor guiding means 34 as well as in the radiation source guiding means 33. In particular, the first position sensor 61 is provided in the position sensor guiding means 34, and a second position sensor 62 is provided in the radiation source guiding means 33, especially behind the radiation source 31. Further, the applicator 30 comprises tissue sensor guiding means 32 provided for housing at least one of a first and second tissue sensors 51, 52. In particular, the first and/or second tissue sensor 51, 52 can be arranged at or at least proximate to a distal end 30a of the applicator 30. Thereby, a first tissue sensor 51 can be provided in the form of a pH sensor 51a. Alternatively, the first tissue sensor 51 can be provided in the form of an O₂ sensor 51b, an optical spectroscopy sensor 51c or an optical coherence tomography sensor 51d. A second tissue sensor 52 can be provided in the form of an ultrasonic probe 52. In other words, Fig. 2 illustrates one embodiment incorporating radiation source 31(e.g. x-ray source 31a or radioactive radiation source 31b), US probe 52 or alternative tissue sensor 51, FBG 61, and EM coil 62. One or several coils 62 for EM tracking can be placed e.g. behind the source, as shown, but also around an optical fiber 61, in any tubing 32, 33, 34, or in an applicator which may be of e.g. needle, tubing or balloon type.

Generally, the first and/or second tissue sensing means 51, 52 as well as the first and/or second position sensing means 61, 62 can be arranged within the guiding means 32, 33, 34, at least partially adjacent to the radiation source 31, especially at or at least proximate to the distal end 30a of the guiding means 32, 33, 34. The guiding means 32, 33, 34 can be provided in the form of a channel-like structure, especially a solid multi-channel arrangement, so that generally, for each sensor, a specific channel-like or tube-like guiding device can be provided. Alternatively, several or all sensors can be provided within one specific channel of the guiding means. This means that each sensor can be provided in a specific channel, but at least two or even all sensor can be provided in one specific channel also, when appropriate. The channel-like arrangement provides a catheter or an applicator in the form of a needle, tubing or balloon design. In other words, basically, the brachytherapy probe can include several sensing means independently of its specific design, and independently of the number of channels, i.e., a single channel may be sufficient.

Fig. 4 shows a schematic drawing of an applicator according to a third embodiment, incorporating means for localization as well as two means for monitoring. The radiation source 31 is placed as shown in Fig. 1 and 2, that is to say, within radiation source guiding means 33. The means for localization are provided in the form of the first position sensor 61 in the position sensor guiding means 34. Further, the applicator 30 comprises tissue sensor guiding means 32 provided for housing a first and a second tissue sensors 51, 52.
Thereby, as mentioned in context with Fig. 3, the first tissue sensor 51 can be provided in the form of a pH sensor 51a or an O₂ sensor 51b, an optical spectroscopy sensor 51c or an optical coherence tomography sensor 51d. The second tissue sensor 52 is provided in the form of an ultrasonic probe 52, both sensors 51, 52 being arranged within tissue sensor guiding means 32. It shall be understood that tissue sensors 51, 52 alternatively can be arranged within position sensor guiding means 34 and position sensors 61, 62 alternatively can be arranged within tissue sensor guiding means 32. The tissue sensors 51, 52 alternatively can be arranged in radiation source guiding means 33 also.

In summary, in brachytherapy where position information relating to a radiation source is to be generated, a guidance system is adapted to acquire and process position data or position and tissue data, so that high-precision interventional radiotherapy can be carried out, especially according to a dose plan and for intra-fraction monitoring for a therapy plan and/or adaptive re-planning. The data can be stored and used to refine future treatment plans and for future correlation with long-term treatment outcome, especially in context with low energy brachytherapy.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, sensing unit or other unit may fulfill the functions of several items recited in the claims. The mere fact that certain measures are recited in mutually different dependent claims does not indicate that a combination of these measures cannot be used to advantage.

It is noted that the proposed solution according to the above embodiments can be implemented at least partially in software modules at the relevant functional blocks of Fig. 1. The resulting computer program product may comprise code means for causing a computer to carry out the steps of the above procedures of functions of Fig. 1. Hence, the procedural steps are produced by the computer program product when run on the computer.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope thereof.

In brachytherapy where position information relating to a radiation source is to be generated, a guidance system is adapted to acquire and process position data or position and tissue data, so that high-precision interventional radiotherapy can be carried out, especially according to a dose plan and for intra-fraction monitoring for a therapy plan or adaptive re-planning. The data can be stored and used to refine future treatment plans and for future correlation with long-term treatment outcome, especially in context with low energy brachytherapy.

## Claims

1. An apparatus (30) for interventional brachytherapy for generating data to be used directly for therapy and/or for therapy planning, said apparatus (30) comprising:
- guiding means (32, 33, 34) for providing a channel-like arrangement to be navigated within a body in order to irradiate tissue;
- a radiation source (31) for irradiating tissue, said radiation source (31) being arranged on said guiding means (32, 33, 34); and
- first position sensing means (61) for generating position data related to the position of said radiation source (31), said first position sensing means (61) being arranged to provide the position data to a position control device (22) and/or power control device (21) of a brachytherapy system in order to position said radiation source (31) within the body,
wherein said first position sensing means (61) comprises an optical fiber and at least one optical sensor for position detection;
- first tissue sensing means (51) for generating tissue data related to tissue characteristics, said first tissue sensing means (51) being arranged to provide the tissue data to said position control device (22) and/or said power control device (21) of said brachytherapy system in order to analyze tissue.

2. The apparatus according to claim 1, said first tissue sensing means (51) being composed of at least one sensor of the group comprising a pH sensor (51a), an O₂ sensor (51b), an optical spectroscopy sensor (51c) and an optical coherence tomography sensor (51d).

3. The apparatus according to claim 1, said first position sensing means (61) including its optical fiber being arranged within said guiding means (32, 33, 34), at least partially adjacent to said radiation source (31), said first position sensing means (61) further being arranged at or at least proximate to the distal end (30a) of said guiding means (32, 33, 34);
said apparatus (30) further comprising second position sensing means (62) in the form at least one electromagnetic (EM) tracking sensor, said second position sensing means (62) being arranged within said guiding means (32, 33, 34),
wherein said first (61) and second position sensing means (62) are arranged in such a way that both position data of said radiation source (31) and the position data of the distal end (30a) can be provided, said first (61) and second position sensing means (62) providing different spatial resolution, either in the range of cm, mm, or sub-mm accuracy.

4. The apparatus according to claim 1, said first tissue sensing means (51) being arranged within said guiding means (32, 33, 34), at least partially adjacent to said radiation source (31), said first tissue sensing means (51) further being arranged at or at least proximate to the distal end (30a) of said guiding means (32, 33, 34);
said apparatus (30) further comprising second tissue sensing means (52) in the form of at least one ultrasonic (US) probe, said second tissue sensing means (52) being arranged within said guiding means (32, 33, 34),
wherein said first (51) and second tissue sensing means (52) are arranged in such a way that both tissue data of tissue to be irradiated by said radiation source (31) and tissue data of tissue encompassing the tissue to be irradiated can be provided, said first (51) and second tissue sensing means (52) providing different resolution.

5. The apparatus according to claim 1, said radiation source (31) being arranged within a radiation source guiding means (33), and said first position sensing means (61) being arranged within a position sensor guiding means (34) which is provided adjacent to said radiation source guiding means (33), and said first tissue sensing means (51) being arranged within a tissue sensor guiding means (32) which is provided adjacent to said radiation source guiding means (33).

6. The apparatus according to claim 2,
said first tissue sensing means (51) being at least composed of an optical spectroscopy sensor (51 c) using visible to infrared (IR) wavelength, excitation light being provided from a light source ex vivo via first optical fiber means, and reflected light being provided via said first or via second optical fiber means;
wherein said apparatus is connected to a grating spectrometer ex vivo for analyzing reflected light.

7. The apparatus according to claim 4,
said second tissue sensing means (52) being provided in the form of at least two chips for ultrasonic (US) detection, said chips being mounted around said radiation source (31) and/or around said optical fiber and/or along said guiding means (32, 33, 34).

8. An apparatus according to claim 1, wherein the guiding means are arranged in an applicator comprising a radiation source guiding means (33) for housing a radiation source (31), a position sensor guiding means (34) for housing position sensing means (61, 62), and a tissue sensor guiding means (32) for housing tissue sensing means (51, 52), each of said guiding means (32, 33, 34) being provided in the form of a channel-like guiding means (32, 33, 34) being arranged adjacent to at least one other of said guiding means (32, 33, 34), so that said channel-like arrangement is provided with each channel-like guiding means (32, 33, 34) being arranged adjacent to at least one other of said channel-like guiding means (32, 33, 34),
wherein said applicator device is arranged for providing position data of a radiation source (31) and/or tissue data of tissue to be treated.

9. The applicator device according to claim 8, wherein said applicator device is arranged for housing, in said position sensor guiding means (34), an optical fiber for providing excitation radiation to the distal end of said position sensor guiding means (34) and/or reflected radiation to the proximal end of said position sensor guiding means (34), and
wherein said applicator device is arranged for housing, in said tissue sensor guiding means (32), an optical fiber for providing excitation radiation to the distal end of said tissue sensor guiding means (32) and/or reflected radiation to the proximal end of said tissue sensor guiding means (32).

10. A brachytherapy system for interventional radiotherapy for generating position information and/or tissue information to be processed directly for therapy and/or to be processed for therapy planning in order to apply a radiation dose according to a dose plan (40), said system comprising:
a. a brachytherapy apparatus (20) comprising a power control device (21) and a position control device (22);
b. an apparatus (30) according to claim 1;
wherein said first position sensing means (61) and said tissue sensing means (51) provide position data and tissue data to the brachytherapy apparatus (20), the power control device (21) being in communication with said radiation source (31).

11. A brachytherapy system according to claim 10, wherein said radiation source (31) is in communication with said brachytherapy apparatus (20) by means of a first connection (10), said first position sensing means (61) and said tissue sensing means (51, 52) being in communication with each other via a second connection (11), said first position sensing means (61) and said tissue sensing means (51, 52) being in communication with said brachytherapy apparatus (20) via a third connection (12).

12. A storage medium storing a computer program program
product comprising code means for producing the following steps when run on a computing device:
- navigating an apparatus (30) with guiding means (32, 33, 34) within a body for providing a radiation source (31) generating radiation in order to treat tissue;
- generating position data by means of position sensing means (61) having an optical fiber and at least one position sensor;
- generating tissue data related to tissue characteristics by means of tissue sensing means (51), and
- providing the position data and the tissue data to a brachytherapy apparatus (20) in order to control power and/or position of said radiation source (31)..

## Patentansprüche

1. Gerät (30) für eine interventionelle Brachytherapie zum Erzeugen von Daten, die direkt für die Therapie und/oder die Therapieplanung zu verwenden sind, wobei das genannte Gerät (30) Folgendes umfasst:
- Führungsmittel (32, 33, 34) zum Bereitstellen einer kanalartigen Anordnung, die innerhalb eines Körpers zu navigieren ist, um Gewebe zu bestrahlen;
- eine Strahlungsquelle (31) zum Bestrahlen von Gewebe, wobei die genannte Strahlungsquelle (31) an dem genannten Führungsmittel (32, 33, 34) angeordnet ist; und
- ein erstes Positionserfassungsmittel (61) zum Erzeugen von Positionsdaten in Bezug auf die Position der genannten Strahlungsquelle (31), wobei das genannte erste Positionserfassungsmittel (61) dafür vorgesehen ist, die Positionsdaten einer Positionssteuerungsvorrichtung (22) und/oder Leistungssteuerungsvorrichtung (21) eines Brachytherapiesystems bereitzustellen, um die Strahlungsquelle (31) innerhalb des Körpers zu positionieren,
wobei das genannte erste Positionserfassungsmittel (61) eine optische Faser und mindestens einen optischen Sensor zur Positionserkennung umfasst;
- ein erstes Gewebeerfassungsmittel (51) zum Erzeugen von Gewebedaten in Bezug auf Gewebeeigenschaften, wobei das genannte erste Gewebeerfassungsmittel (51) dafür vorgesehen ist, die Gewebedaten der genannten Positionssteuerungsvorrichtung (22) und/oder der genannten Leistungssteuerungsvorrichtung (21) des genannten Brachytherapiesystems bereitzustellen, um das Gewebe zu analysieren.

2. Gerät nach Anspruch 1, wobei das genannte erste Gewebeerfassungsmittel (51) zusammengesetzt ist aus mindestens einem Sensor der Gruppe bestehend aus einem pH-Sensor (51a), einem O₂-Sensor (51b), einem optischen Spektroskopiesensor (51c) und einem optischen Kohärenztomographiesensor (51 d).

3. Gerät nach Anspruch 1, wobei das genannte erste Positionserfassungsmittel (61) einschließlich seiner optischen Faser innerhalb des genannten Führungsmittels (32, 33, 34), zumindest teilweise angrenzend an die genannte Strahlungsquelle (31), angeordnet ist, wobei das genannte erste Positionserfassungsmittel (61) weiterhin an oder mindestens proximal zu dem distalen Ende (30a) des genannten Führungsmittels (32, 33, 34) angeordnet ist;
wobei das genannte Gerät (30) weiterhin ein zweites Positionserfassungsmittel (62) in Form von mindestens einem elektromagnetischen (EM) Trackingsensor umfasst, wobei das genannte zweite Positionserfassungsmittel (62) innerhalb des genannten Führungsmittels (32, 33, 34) angeordnet ist,
wobei das genannte erste (61) und das zweite Positionserfassungsmittel (62) auf derartige Weise angeordnet sind, dass sowohl die Positionsdaten der genannten Strahlungsquelle (31) als auch die Positionsdaten des distalen Endes (30a) bereitgestellt werden können, wobei das erste (61) und das zweite Positionserfassungsmittel (62) eine unterschiedliche räumliche Auflösung, entweder im Genauigkeitsbereich von cm, mm oder sub-mm, liefern.

4. Gerät nach Anspruch 1, wobei das genannte erste Gewebeerfassungsmittel (51) innerhalb des genannten Führungsmittels (32, 33, 34), zumindest teilweise angrenzend an die genannte Strahlungsquelle (31), angeordnet ist, wobei das genannte erste Gewebeerfassungsmittel (51) weiterhin an oder mindestens proximal zu dem distalen Ende (30a) des genannten Führungsmittels (32, 33, 34) angeordnet ist;
wobei das genannte Gerät (30) weiterhin ein zweites Gewebeerfassungsmittel (52) in Form von mindestens einer Ultraschallsonde (US) umfasst, wobei das genannte zweite Gewebeerfassungsmittel (52) innerhalb des genannten Führungsmittels (32, 33, 34) angeordnet ist,
wobei das genannte erste (51) und das zweite Gewebeerfassungsmittel (52) auf derartige Weise angeordnet sind, dass sowohl die Gewebedaten von Gewebe, das durch die genannte Strahlungsquelle (31) zu bestrahlen ist, als auch Gewebedaten von Gewebe, das das zu bestrahlende Gewebe umschließt, bereitgestellt werden können, wobei das erste (51) und das zweite Gewebeerfassungsmittel (52) eine unterschiedliche Auflösung liefern.

5. Gerät nach Anspruch 1, wobei die genannte Strahlungsquelle (31) innerhalb eines Strahlungsquellenführungsmittels (33) angeordnet ist und das genannte erste Positionserfassungsmittel (61) innerhalb eines Positionssensorführungsmittels (34) angeordnet ist, das angrenzend an das genannte Strahlungsquellenführungsmittel (33) vorgesehen ist, und wobei das genannte erste Gewebeerfassungsmittel (51) innerhalb eines Gewebesensorführungsmittels (32) angeordnet ist, das angrenzend an das genannte Strahlungsquellenführungsmittel (33) vorgesehen ist.

6. Gerät nach Anspruch 2,
wobei das erste Gewebeerfassungsmittel (51) mindestens zusammengesetzt ist aus einem optischen Spektroskopiesensor (51 c), der sichtbare bis Infrarot- (IR) Wellenlänge nutzt, wobei das Anregungslicht von einer Lichtquelle ex vivo über erste optische Fasermittel bereitgestellt wird und wobei reflektiertes Licht über die genannten ersten oder über zweite optische Fasermittel bereitgestellt wird;
wobei das genannte Gerät mit einem Gitterspektrometer ex vivo zum Analysieren des reflektierten Lichts verbunden ist.

7. Gerät nach Anspruch 4,
wobei das genannte zweite Gewebeerfassungsmittel (52) in Form von mindestens zwei Chips zur Ultraschallerkennung (US) vorgesehen ist, wobei die genannten Chips um die genannte Strahlungsquelle (31) herum und/oder um die genannte optische Faser herum und/oder entlang des genannten Führungsmittels (32, 33, 34) montiert sind.

8. Gerät nach Anspruch 1, wobei die Führungsmittel in einem Applikator angeordnet sind, der ein Strahlungsquellenführungsmittel (33) zum Aufnehmen einer Strahlungsquelle (31), ein Positionssensorführungsmittel (34) zum Aufnehmen von Positionserfassungsmitteln (61, 62) und ein Gewebesensorführungsmittel (32) zum Aufnehmen von Gewebeerfassungsmitteln (51, 52) umfasst, wobei jedes der genannten Führungsmittel (32, 33, 34) in Form eines kanalartigen Führungsmittels (32, 33, 34), das angrenzend an mindestens ein anderes der genannten Führungsmittel (32, 33, 34) angeordnet ist, bereitgestellt wird, so dass die genannte kanalartige Anordnung mit jedem kanalartigen Führungsmittel (32, 33, 34) angrenzend an mindestens ein anderes der genannten kanalartigen Führungsmittel (32, 33, 34) bereitgestellt wird,
wobei die genannte Applikatorvorrichtung vorgesehen ist, um Positionsdaten einer Strahlungsquelle (31) und/oder Gewebedaten von zu behandelndem Gewebe zu liefern.

9. Applikatorvorrichtung nach Anspruch 8, wobei die genannte Applikatorvorrichtung vorgesehen ist, um in dem genannten Positionssensorführungsmittel (34) eine optische Faser zum Bereitstellen von Anregungsstrahlung für das distale Ende des genannten Positionssensorführungsmittels (34) und/oder von reflektierter Strahlung für das proximale Ende des genannten Positionssensorführungsmittels (34) aufzunehmen, und
wobei die genannte Applikatorvorrichtung vorgesehen ist, um in dem genannten Gewebesensorführungsmittel (32) eine optische Faser zum Bereitstellen von Anregungsstrahlung für das distale Ende des genannten Gewebesensorführungsmittels (32) und/oder von reflektierter Strahlung für das proximale Ende des genannten Gewebesensorführungsmittels (32) aufzunehmen.

10. Brachytherapiesystem für eine interventionelle Radiotherapie zum Erzeugen von Positionsinformationen und/oder Gewebeinformationen, die direkt für die Therapie zu verarbeiten sind und/oder für die Therapieplanung zu verarbeiten sind, um eine Strahlendosis entsprechend einem Dosisplan (40) zu verabreichen, wobei das genannte System Folgendes umfasst:
a. ein Brachytherapiegerät (20) mit einer Leistungssteuerungsvorrichtung (21) und einer Positionssteuerungsvorrichtung (22);
b. ein Gerät (30) nach Anspruch 1;
wobei das genannte erste Positionserfassungsmittel (61) und das genannte Gewebeerfassungsmittel (51) Positionsdaten und Gewebedaten an das Brachytherapiegerät (20) liefern, wobei die Leistungssteuerungsvorrichtung (21) in Kommunikationsverbindung mit der genannten Strahlungsquelle (31) steht.

11. Brachytherapiesystem nach Anspruch 10, wobei die genannte Strahlungsquelle (31) über eine erste Verbindung (10) in Kommunikationsverbindung mit dem genannten Brachytherapiegerät (20) steht, wobei das genannte erste Positionserfassungsmittel (61) und die genannten Gewebeerfassungsmittel (51, 52) über eine zweite Verbindung (11) in Kommunikationsverbindung miteinander stehen, wobei das genannte erste Positionserfassungsmittel (61) und die genannten Gewebeerfassungsmittel (51, 52) über eine dritte Verbindung (12) in Kommunikationsverbindung mit dem genannten Brachytherapiegerät (20) stehen.

12. Speichermedium zum Speichern eines Computerprogrammprodukts mit Codemitteln zum Erzeugen der folgenden Schritte, wenn es auf einer Computervorrichtung ausgeführt wird:
- Navigieren eines Geräts (30) mit Führungsmitteln (32, 33, 34) innerhalb eines Körpers zur Bereitstellung einer Strahlungsquelle (31), die Strahlung zur Behandlung von Gewebe erzeugt;
- Erzeugen von Positionsdaten mithilfe von Positionserfassungsmitteln (61), die eine optische Faser und mindestens einen Positionssensor haben;
- Erzeugen von Gewebedaten in Bezug auf Gewebeeigenschaften mithilfe von Gewebererfasssungsmitteln (51), und
- Bereitstellen der Positionsdaten und der Gewebedaten an ein Brachytherapiegerät (20), um die Leistung und/oder die Position der genannten Strahlungsquelle (31) zu steuern.

## Revendications

1. Appareil (30) pour curiethérapie interventionnelle pour générer des données destinées à être utilisées directement pour la thérapie et/ou pour la planification de thérapie, ledit appareil (30) comprenant :
- des moyens de guidage (32, 33, 34) pour fournir un agencement en forme de canal destiné à être déplacé à l'intérieur d'un corps afin d'irradier un tissu ;
- une source de radiation (31) pour irradier un tissu, ladite source de radiation (31) étant agencée sur lesdits moyens de guidage (32, 33, 34) ; et
- un premier moyen de détection de position (61) pour générer des données de position connexes à la position de ladite source de radiation (31), ledit premier moyen de détection de position (61) étant agencé pour fournir les données de position à un dispositif de commande de position (22) et/ou un dispositif de commande de puissance (21) d'un système de curiethérapie afin de positionner ladite source de radiation (31) à l'intérieur du corps,
dans lequel ledit premier moyen de détection de position (61) comprend une fibre optique et au moins un capteur optique pour la détection de position ;
- un premier moyen de détection de tissu (51) pour générer des données de tissu connexes à des caractéristiques de tissu, ledit premier moyen de détection de tissu (51) étant agencé pour fournir les données de tissu audit dispositif de commande de position (22) et/ou audit dispositif de commande de puissance (21) dudit système de curiethérapie afin d'analyser un tissu.

2. Appareil selon la revendication 1, ledit premier moyen de détection de tissu (51) étant composé d'au moins un capteur du groupe comprenant un capteur de pH (51a), un capteur d'O₂ (51b), un capteur spectroscopique optique (51c) et un capteur tomographique de cohérence optique (51d).

3. Appareil selon la revendication 1, ledit premier moyen de détection de position (61) comprenant sa fibre optique étant agencée à l'intérieur desdits moyens de guidage (32, 33, 34), de façon au moins partiellement adjacente à ladite source de radiation (31), ledit premier moyen de détection de position (61) étant en outre agencé à, ou au moins à proximité de, l'extrémité distale (30a) desdits moyens de guidage (32, 33, 34) ;
ledit appareil (30) comprenant en outre un second moyen de détection de position (62) sous forme d'au moins un capteur de suivi électromagnétique (EM), ledit second moyen de détection de position (62) étant agencé à l'intérieur desdits moyens de guidage (32, 33, 34),
dans lequel lesdits premiers (61) et seconds (62) moyens de détection de position sont agencés de manière telle que les données de position de ladite source de radiation (31) ainsi que les données de position de l'extrémité distale (30a) puissent être fournies, lesdits premiers (61) et seconds (62) moyens de détection de position fournissant une résolution spatiale différente, dans la plage de précision de cm, de mm, ou de sous-mm.

4. Appareil selon la revendication 1, ledit premier moyen de détection de tissu (51) étant agencé à l'intérieur desdits moyens de guidage (32, 33, 34), de façon au moins partiellement adjacente à ladite source de radiation (31), ledit premier moyen de détection de tissu (51) étant en outre agencé à, ou au moins à proximité de, l'extrémité distale (30a) desdits moyens de guidage (32, 33, 34) ;
ledit appareil (30) comprenant en outre un second moyen de détection de tissu (52) sous forme d'au moins une sonde ultrasonore (US), ledit second moyen de détection de tissu (52) étant agencé à l'intérieur desdits moyens de guidage (32, 33, 34),
dans lequel lesdits premiers (51) et seconds (52) moyens de détection de tissu sont agencés de manière telle que les données de tissu d'un tissu destiné à être irradié par ladite source de radiation (31) ainsi que les données de tissu d'un tissu englobant le tissu destiné à être irradié puissent être fournies, lesdits premiers (51) et seconds (52) moyens de détection de tissu fournissant une résolution différente.

5. Appareil selon la revendication 1, ladite source de radiation (31) étant agencée à l'intérieur d'un moyen de guidage de source de radiation (33), et ledit premier moyen de détection de position (61) étant agencé à l'intérieur d'un moyen de guidage de capteur de position (34) qui est prévu de façon adjacente audit moyen de guidage de source de radiation (33), et ledit premier moyen de détection de tissu (51) étant agencé à l'intérieur d'un moyen de guidage de capteur de tissu (32) qui est prévu de façon adjacente audit moyen de guidage de source de radiation (33).

6. Appareil selon la revendication 2,
ledit premier moyen de détection de tissu (51) étant au moins composé d'un capteur spectroscopique optique (51c) en utilisant une longueur d'onde visible à infrarouge (IR), de la lumière d'excitation étant fournie à partir d'une source lumineuse ex vivo par l'intermédiaire d'un premier moyen à fibre optique, et de la lumière réfléchie étant fournie par l'intermédiaire dudit premier ou par l'intermédiaire de second moyen à fibre optique ;
dans lequel ledit appareil est connecté à un spectromètre à réseau ex vivo pour analyser de la lumière réfléchie.

7. Appareil selon la revendication 4,
ledit second moyen de détection de tissu (52) étant fourni sous forme d'au moins deux puces pour détection ultrasonore (US), lesdites puces étant montées autour de ladite source de radiation (31) et/ou autour de ladite fibre optique et/ou le long desdits moyens de guidage (32, 33, 34).

8. Appareil selon la revendication 1, dans lequel les moyens de guidage sont agencés dans un applicateur comprenant un moyen de guidage de source de radiation (33) pour loger une source de radiation (31), un moyen de guidage de capteur de position (34) pour loger des moyens de détection de position (61, 62), et un moyen de guidage de capteur de tissu (32) pour loger des moyens de détection de tissu (51, 52), chacun desdits moyens de guidage (32, 33, 34) étant fourni sous forme de moyen de guidage en forme de canal (32, 33, 34) agencé de façon adjacente à au moins un autre desdits moyens de guidage (32, 33, 34), pour que ledit agencement en forme de canal soit pourvu de chaque moyen de guidage en forme de canal (32, 33, 34) agencé de façon adjacente à au moins un autre desdits moyens de guidage en forme de canal (32, 33, 34),
dans lequel ledit dispositif applicateur est agencé pour fournir des données de position d'une source de radiation (31) et/ou des données de tissu d'un tissu destiné à être traité.

9. Dispositif applicateur selon la revendication 8, dans lequel ledit dispositif applicateur est agencé pour loger, dans ledit moyen de guidage de capteur de position (34), une fibre optique pour fournir une radiation d'excitation à l'extrémité distale dudit moyen de guidage de capteur de position (34) et/ou une radiation réfléchie à l'extrémité proximale dudit moyen de guidage de capteur de position (34), et
dans lequel ledit dispositif applicateur est agencé pour loger, dans ledit moyen de guidage de capteur de tissu (32), une fibre optique pour fournir une radiation d'excitation à l'extrémité distale dudit moyen de guidage de capteur de tissu (32) et/ou une radiation réfléchie à l'extrémité proximale dudit moyen de guidage de capteur de tissu (32).

10. Système de curiethérapie pour radiothérapie interventionnelle pour générer des informations de position et/ou des informations de tissu destinées à traitées directement pour la thérapie et/ou destinées à être traitées pour la planification de thérapie afin d'appliquer une dose de radiation selon un plan de dose (40), ledit système comprenant :
a. un appareil de curiethérapie (20) comprenant un dispositif de commande de puissance (21) et un dispositif de commande de position (22) ;
b. un appareil (30) selon la revendication 1,
dans lequel ledit premier moyen de détection de position (61) et ledit moyen de détection de tissu (51) fournissent des données de position et des données de tissu à l'appareil de curiethérapie (20), le dispositif de commande de puissance (21) étant en communication avec ladite source de radiation (31).

11. Système de curiethérapie selon la revendication 10, dans lequel ladite source de radiation (31) est en communication avec ledit appareil de curiethérapie (20) au moyen d'une première connexion (10), ledit premier moyen de détection de position (61) et lesdits moyens de détection de tissu (51, 52) étant en communication les uns avec les autres par l'intermédiaire d'une deuxième connexion (11), ledit premier moyen de détection de position (61) et lesdits moyens de détection de tissu (51, 52) étant en communication avec ledit appareil de curiethérapie (20) par l'intermédiaire d'une troisième connexion (12).

12. Support de stockage stockant un produit programme d'ordinateur comprenant des moyens codes pour produire les étapes suivantes lors de l'exécution sur un dispositif informatique :
- le déplacement d'un appareil (30) avec des moyens de guidage (32, 33, 34) à l'intérieur d'un corps pour fournir une source de radiation (31) générant une radiation afin de traiter un tissu ;
- la génération de données de position en utilisant un moyen de détection de position (61) comportant une fibre optique et au moins un capteur de position ;
- la génération de données de tissu connexes à des caractéristiques de tissu en utilisant un moyen de détection de tissu (51), et
- la fourniture des données de position et des données de tissu à un appareil de curiethérapie (20) afin de commander la puissance et/ou la position de ladite source de radiation (31).
